# EUROPEAN PATENT APPLICATION

(11) **EP 2 738 557 A1**
(43) Date of publication of application: **04.06.2014**
(21) Application number: 12195271.7
(22) Date of filing: 03.12.2012
(51) Int. Cl.: G01N 33/574

(54) **Organized immune response in cancer**

(71) Applicant: Université Libre de Bruxelles, 1050 Bruxelles (BE)
(72) Inventor: Willard-Gallo, Karen, 1300 Wavre (BE); Gu-Trantien, Chunyan, 92310 Sevres (FR); Garaud, Soizic, 1170 Bruxelles (BE); Buisseret, Laurence, 1180 Bruxelles (BE)
(74) Representative: Pronovem

(57) **Abstract**

A method for prognosticating the response of a solid-tumor cancer comprising the step of measuring the expression of CXCL13 on a tumor sample and of measuring the expression of a (1, 2, 3, 4, 5, 6, or all the 7) gene(s) selected from the group consisting of CD200, FBLN7, ICOS, SGPP2, SH2D1A, TIGIT and PDCD1.

## Description

### Field of the invention

The present invention relates to cancer diagnostic in relation to local immunity.

### Background of the invention and state of the art

Immune cells do not expand and function normally within the tumor microenvironment.

CD8+ cytotoxic T-cells have been considered the key component of effective anti-tumor immunity, although studies now show they frequently fail to function *in vivo* due to a lack of CD4+ T-cell help. Recent investigations into the role of human CD4+ T-cells in anti-tumor immunity have largely focused on the suppressive activities of regulatory T-cells in dampening immune functions and designing therapeutic strategies to relieve this effect.

The detection of an immune signal has been the driving force behind recent promising gene signatures, shown to have both prognostic and predictive value for various cancers.

In breast cancer (BC), a high immune signal has been linked with improved patient outcome in several subtypes (Desmedt, C. et al. Biological processes associated with breast cancer clinical outcome depend on the molecular subtypes. Clin Cancer Res 14, 5158-5165, 2008) .

The signals emanating from these analyses of total tumor tissue reflect a broad spectra of immune response genes and activities of the innate and adaptive immune responses, with some refined to a T-cell metagene, B-cell metagene or specific signaling pathways, including STAT1.

For instance, Ignatiadis, M. *et al.* (Gene Modules and Response to Neoadjuvant Chemotherapy in Breast Cancer Subtypes: A Pooled Analysis. *Journal of clinical oncology : official journal of the American Society of Clinical Oncology,* doi:10.1200/JCO.2011.39.5624 (2012)) have recently shown that a higher immune score in the initial biopsy predicts increased pCR rates after preoperative chemotherapy, with the strongest association observed in the HER2+ and ER-/HER2-.

### Summary of the invention

A first aspect of the present invention is a diagnostic kit comprising tools for measuring CXCL13 gene expression, and tools for measuring the expression of a (1, 2, 3, 4, 5, 6, or all the 7) gene (s) selected from the group consisting of CD200, FBLN7, ICOS, SGPP2, SH2D1A, TIGIT and PDCD1.

Preferably, this kit comprises antibodies specifically recognizing one or more (1, 2, 3 or all the 4) proteins (encoded by the 1, 2, 3 or all the 4 gene(s)) selected from the group consisting of CD200, ICOS, PDCD1 and TIGIT protein(s) and further comprising antibodies specifically recognizing CD4 protein (in order to measure expression of the corresponding proteins, preferably in CD4 lymphocytes, such as in FACS co-labelling of CD4 and one or more of CD200, ICOS, PDCD1 and TIGIT proteins).

Preferably, this kit further comprises tools for measuring the expression of (TH1) genes selected from the group consisting of CXCL9, IFNG, PMAIP1, IL12RB2, CD38 and CTLA4 gene(s).

A related aspect is the use of this kit for the diagnostic (prognosis) of patients having a solid-tumor cancer selected from the group consisting of breast cancer, pancreatic cancer, prostate cancer, colorectal cancer, lung cancer, hepatocellular carcinoma and renal cell carcinoma, preferably breast cancer.

Another related aspect is the use of this kit for monitoring the immune response of a patient towards a solid-tumor cancer selected from the group consisting of breast cancer, pancreatic cancer, prostate cancer, colorectal cancer, lung cancer, hepatocellular carcinoma and renal cell carcinoma, preferably breast cancer.

Another related aspect is the use of this kit for monitoring anticancer treatment of a patient suffering from a solid-tumor cancer selected from the group consisting of breast cancer, pancreatic cancer, prostate cancer, colorectal cancer, lung cancer, hepatocellular carcinoma and renal cell carcinoma, preferably breast cancer.

Another related aspect is an isolated human (Tfh) CD4-positive lymphocyte having high expression level of PD-1 (PDCD1) and of CD200 proteins.

Preferably, this lymphocyte has low or no expression of CD26 protein (and an intermediate CD38 level).

Still another related aspect is a method for prognosticating the outcome of a solid-tumor cancer selected from the group consisting of breast cancer, pancreatic cancer, prostate cancer, colorectal cancer, lung cancer, hepatocellular carcinoma and renal cell carcinoma, preferably breast cancer, comprising the step of homogeneizing in vitro a (part of a) tumor sample (from this solid-tumor cancer); performing a centrifugation of the said homogenized sample; applying the obtained supernatant to a culture of CD4-positive (CD4+) reporter cells and measuring the effects on proliferation of the said CD4-positive cells in response to stimulation (and preferably of measuring the expression on (a part of) the whole tumor sample (from this solid-tumor cancer) of CXCL13 gene and of a (1, 2, 3, 4, 5, 6, or all the 7) gene(s) selected from the group consisting of CD200, FBLN7, ICOS, SGPP2, SH2D1A, TIGIT and PDCD1; and/or of assessing the presence of tissular lymphoid structures containing specific B and specific T cells, such as CD4-positive follicular helper T cells (Tfh) and/or Bm3 and/or Bm4 B cells within the tumor sample (from this solid-tumor cancer; preferably assessing the presence of CD4+ Tfh (such as CD200+ and PDCD1+ and/or CD26-), and of Bm3 and of Bm4 cells)).

Still another related aspect is a method (an alternative method) for prognosticating the outcome of a solid-tumor cancer selected from the group consisting of breast cancer, pancreatic cancer, prostate cancer, colorectal cancer, lung cancer, hepatocellular carcinoma and renal cell carcinoma, preferably breast cancer, comprising the step of measuring the expression on (a part of) a whole tumor sample (from this solid-tumor cancer) of CXCL13 gene and of a (1, 2, 3, 4, 5, 6, or all the 7) gene(s) selected from the group consisting of CD200, FBLN7, ICOS, SGPP2, SH2D1A, TIGIT and PDCD1 (and preferably of further assessing the presence of tissular lymphoid structures containing specific B and specific T cells, such as CD4-positive follicular helper T cells (Tfh) and/or Bm3 and/or Bm4 B cells (preferably assessing the presence of CD4+ Tfh (such as CD200+ and PDCD1+ and/or CD26-), and of Bm3 and of Bm4 cells) within (a part of) the tumor sample (from this solid-tumor cancer); and/or of homogeneizing in vitro (a part of) the tumor sample (from this solid-tumor cancer); performing a centrifugation of this homogenized sample; applying the obtained supernatant to a culture of CD4+ reporter cells and measuring the effects on proliferation of the said CD4-positive cells in response to stimulation).

Still another related aspect is a method (an alternative method) for prognosticating the outcome of a solid-tumor cancer selected from the group consisting of breast cancer, pancreatic cancer, prostate cancer, colorectal cancer, lung cancer, hepatocellular carcinoma and renal cell carcinoma, preferably breast cancer, comprising the step of assessing the presence of tissular lymphoid structures containing specific B and specific T cells, such as CD4-positive follicular helper T cells (Tfh) and/or Bm3 and/or Bm4 B cells (preferably assessing the presence of CD4+ Tfh(such as CD200+ and PDCD1+ and/or CD26-), and of Bm3 and of Bm4 cells) within a (part of a) tumor sample (from this solid-tumor cancer); (and possibly of homogeneizing in vitro a tumor sample (from this solid-tumor cancer); performing a centrifugation of this homogenized sample; applying the obtained supernatant to a culture of CD4+ reporter cells and measuring the effects on proliferation of the said CD4-positive cells in response to stimulation and/or of measuring the expression on a whole tumor sample (from this solid-tumor cancer) of CXCL13 gene and of a (1, 2, 3, 4, 5, 6, or all the 7) gene(s) selected from the group consisting of CD200, FBLN7, ICOS, SGPP2, SH2D1A, TIGIT and PDCD1).

Advantageously, in these (prognosticating) methods, overexpression of CXCL13 gene and of a (1, 2, 3, 4, 5, 6, or all the 7) gene(s) selected from the group consisting of CD200 (especially measures on CD4+ lymphocytes), FBLN7, ICOS, SGPP2, SH2D1A, TIGIT and PDCD1 in the tumor sample is associated with a good prognostic, as well as the presence of tissular lymphoid structures containing specific B (Bm3 and/or Bm4) and specific T cells (Tfh; such as CD200+ and PDCD1+ and/or CD26-), and/or of rapidly growing reporters CD4+ cells.

Preferably, in these methods the measure of the expression of the CD200, ICOS and PDC1 and/or TIGIT genes is performed on CD4-positive cells present in the tumor sample.

More preferably, in these methods the measure of the expression of CD200, ICOS, PDCD1 and/or TIGIT genes is performed in flow cytometry on CD4-positive lymphocytes freshly isolated from the tumor sample.

Preferably, in these methods the measure of the expression of CD200 gene is performed in dual labelling with PDCD1 protein (on CD4+ lymphocytes freshly isolated from the tumor and/or labelled).

Preferably, in these methods, the expression level of the genes is measured by comparison with the gene expression level of CD4+ cells from an ex vivo sample of peripheral blood from the patient affected with the solid tumor cancer.

A related aspect of the present invention is a method for measuring primary tumor aggressivity and/or for identifying the presence of immunosuppressant factors in tumors comprising the steps of:
- homogeneizing in vitro a tumor sample,
- performing a centrifugation of the said homogenized sample,
- applying the obtained supernatant to a culture of CD4+ reporter cells and
- measuring the effects on proliferation of the said CD4-positive cells upon stimulation,
wherein the said primary tumor is from a cancer selected from the group consisting of breast cancer, pancreatic cancer, prostate cancer, colorectal cancer, lung cancer, hepatocellular carcinoma and renal cell carcinoma, preferably breast cancer.

Preferably, in this method (for measuring primary tumor aggressivity and/or for identifying the presence of immunosuppressant factors in tumors), reduced CD4+ lymphocyte proliferation is (positively) correlated with aggressivity of the primary tumor and/or with the presence of immunosuppressant factors in the primary tumor and/or with an impaired outcome.

Preferably, this method (for measuring primary tumor aggressivity and/or for identifying the presence of immunosuppressant factors in tumors) further comprises the step of adding anti-CD3 and anti-CD28 antibodies to the obtained supernatant to be applied to the culture of CD4+ reporter cells.

A related aspect of the present invention is CXCL13 protein for use in the treatment of solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein a low (or no) expression of the genes and/or no tissular lymphoid structure and/or impaired growth of CD4+ reporter cells is measured by the prognostic method(s) of the present invention.

Preferably, this CXCL13 protein for use in the treatment of solid tumor cancer is tumor targeted and/or directed.

A related aspect of the present invention is Anti PD1 (PDCD1) antibodies for use in the treatment of a solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein a moderate to high expression of the genes and/or tissular lymphoid structures and/or less impaired growth and responsiveness of CD4+ reporter cells is measured by the prognostic method(s) of the present invention.

A related aspect of the present invention is Anti CTLA4 antibodies for use in the treatment of a solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein moderate to high expression of the genes and/or tissular lymphoid structure and/or less impaired growth and responsiveness of CD4+ reporter cells is measured by the prognostic method(s) of the present invention.

### Brief description of the figures and tables

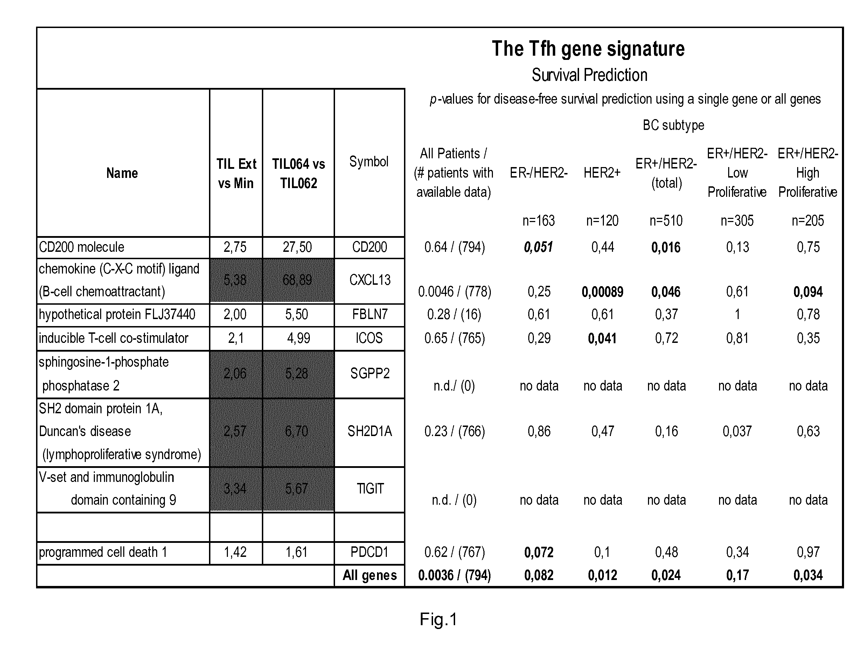

Figure 1: overview of the Tfh gene signature as in the present invention. The genes of the Tfh signatures are associated to their relative expression pattern in maximally infiltrated tumors vs minimally infiltrated tumors (Ext vs Min; Til 064 vs TIL 062). Same tendency was obtained by comparing TIL vs peripheral blood. The expression level of these genes was then correlated to the survival data of patients further classified in breast cancer subtypes. Of interest is the fact that overexpression of CD200 on a whole tumor is a worse prognosis in ER-/HER2- patients, but a good prognosis for the other subgroups of patients, especially in ER+/HER2-, a fact attributable to CD200 expression by some non-lymphocytes tumors cells. However, high expression of CD200 on (CD4+)lymphocytes is consistently associated to a good prognosis.
From these data, the whole Tfh signature, especially taking into account the puzzling effect of CD200, outperforms the prognosis deduced from the expression level of one gene of this signature, especially on HER2- (ER+ or ER-) patients.

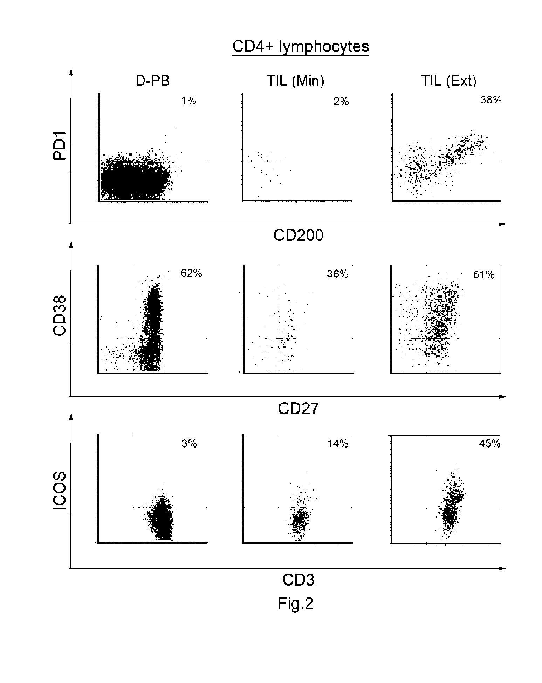

Figure 2 Tfh gene expression in CD4+ TIL from extensive versus minimally-infiltrated BC. Dot plots showing surface expression of Tfh markers on CD4+ TIL from extensive and minimally-infiltrated tumors analyzed using flow cytometry and compared to CD4+ T-cells from D-PB as a control.

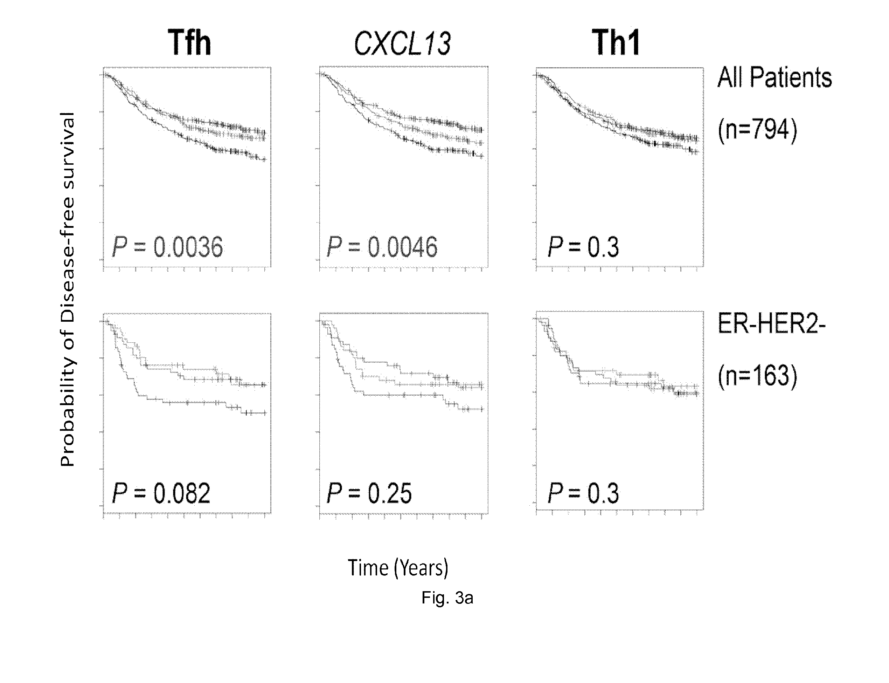

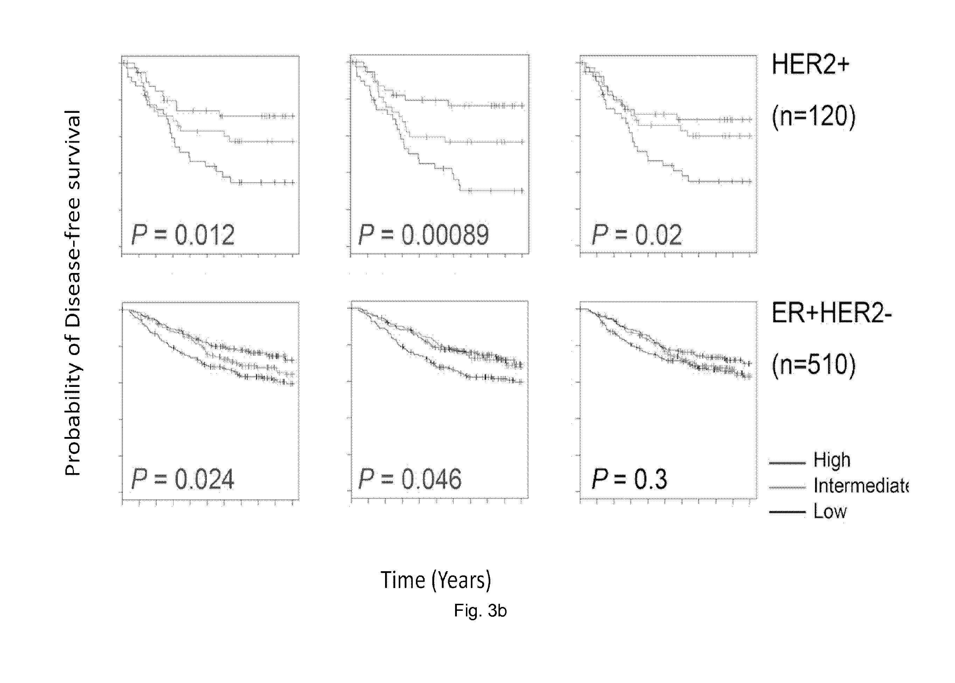

Figure 3 The Tfh signature is linked with positive clinical outcome in BC. The Tfh (including CXCL13 as a single gene) was derived and tested on public microarray datasets from 794 primary systemically untreated BC patients for 10-year DFS. Kaplan-Meier survival curves (probability of survival, from 0.0 to 1.0 in function of time; from 0 year to 10 years) were generated for the total patient population and three major BC subsets: ER-/HER2-, HER2+, ER+/HER2-. Gene expression levels are shown: the low expression curves always correspond to the lowest probability of disease-free survival, intermediate expression appear in grey and corresponds to intermediate disease-free survival probability, and high expression always corresponds to the highest disease-free survival.

### Detailed description of the invention

The inventors have found that, although cancer development reflects the ultimate failure of anti-tumor immunity, it does not necessarily signify a wholly ineffective immune response.

The inventors have characterized CD4⁺ T lymphocytes infiltrating primary human breast tumors (TIL), analyzed T cell signaling pathways influenced by the tumor microenvironment, correlated differential gene expression with the presence of a specific CD4⁺ T cell subset(s) and identified a profile predictive of positive patient outcome.

The inventors have chosen to rely on rapid and enzymatic-free isolation of CD4+ tumor infiltrating lymphocytes (TIL) without *in vitro* expansion. Although such approach is much more difficult to run in practice, this approach was found by the inventors as necessary to accurately reflect the *in situ* state.

Specifically, molecular profiling of CD4⁺ T cells isolated from the primary tumor (TIL), axillary lymph node and peripheral blood of breast cancer (BC) patients demonstrated that the CD4⁺ TIL contain all Th subsets and reflect an activated, effector memory phenotype.

Although all Th subsets are present in the tumor, the inventors have found that they do not contribute equally, with more extensively-infiltrated tumors distinguished by higher expression of Tfh, Th1 and T cell activation markers. The inventors further demonstrated that the TCR/CD3-directed antigen response pathway was significantly suppressed in activated CD4⁺ TIL in association with restricted Th cytokine and chemokine production.

The inventors have observed that patients whose primary tumors have extensive lymphoid infiltrates (characterized by tertiary lymphoid structures (TLS) and/or a germinal center (GC)) have a better clinical outcome than those with minimal infiltration.

In other words, these data establish that the position and prevalence of specific CD4⁺ T cells within the tumor dictate their responsiveness. Further investigation revealed that some tumors successfully sequester the majority of infiltrating leukocytes in organized tertiary lymphoid structures (TLS). Thus, more extensively-infiltrated tumors were shown to be less immunosuppressive, have a higher frequency of TLS and elevated expression of a positive prognostic CD4⁺ Tfh cell signature.

The inventors have mirrored the extensive lymphoid infiltrate by direct quantification of a signature established by measuring the expression level of at least CXCL13 gene and of one or more gene(s) selected from the group consisting of CD200, FBLN7, ICOS, SGPP2, SH2D1A, TIGIT and PDCD1 in a (whole) tumor sample, and/or by histological methods and/or by measuring the abundance of PDCD1 and/or CD200 and/or ICOS positive CD4+ T lymphocytes in the tumor.

PD-1^{hi} Th cells were recently shown to be efficient CXCL13 producers and although PD-1 (PDCD1) expression is generally considered a sign of T-cell exhaustion, the inventors have shown that the measure of CXCL13 expression, in addition to the measure of expression of (at least one) genes selected from the group consisting of CD200, FBLN7, ICOS, SGPP2, SH2D1A, TIGIT and PDCD1, in a tumor is a reliable reflection of tumors extensively-infiltrated by lymphocytes, which rather represents a good prognostic.

In addition, the inventors have deduced that factors present in the tumor micro-environment directly inhibit localized immune responses.
In other words, the inventors have found that extensively-infiltrated tumors, such as tumors comprising (detectable amounts of) specific B cell subpopulations (preferably Bm3 and/or Bm4 cells) and/or associated with detectable amounts of) specific T cells (such as CD4-positive follicular helper T cells) are less immunosuppressive, have a higher frequency of tertiary lymphoid structures (TLS) and enhanced expression of a positive prognostic CD4+ Tfh cell signature. CXCL13-producing CD4+ Tfh TIL play a key role in TLS formation, thereby these cells help to create a haven in the tumor mass where effective and durable anti-tumor immune responses can be generated.

At the base of the invention is the deduction by the inventors that some patients build an organized immune response against a tumor, while others do not. However, comprehensive characterization and quantification of lymphocyte subpopulations infiltrating tumors has been hampered by their extremely low absolute numbers, the need to enzymatically digest fresh tumor tissue, and the semiquantitative nature of immunohistochemistry (IHC) on tissue sections.

Despite the difficulties of the approach, the inventors analyzed freshly resected invasive breast tumor tissue from 70 untreated patients, which have been rapidly mechanically dissociated into a single cell homogenate for immediate *ex vivo* assessment using flow cytometry, qRT-PCR and/or gene expression arrays.

The distribution of infiltrating CD45+ cells included a majority of T-cells (±75%;range=60-90%) together with <20% B-cells, <10% monocytes, <5% NK- or NKT-cells. Among the CD3+ T-cells, the CD4+ subset is consistently the principal component (mean=53% in 35 tumors), outnumbering CD8+ T-cells and present both at a higher frequency and ratio than in ipsilateral non-adjacent normal breast tissue (mean=38% in 31 samples).

An image of the CD4+ Tumor infiltrating lymphocytes (TIL) from a patient in their native state was produced by comparing global gene expression profiles of TIL with CD4+ T-cells from their axillary lymph node (LN) and peripheral blood (PB) (P-PB), as well as PB from healthy donors (D-PB). Notably, CD38, TNFRSF18 (GITR), CD200, CXCL13, ICOS and PDCD1 were among the overexpressed marker genes in CD4+ TIL from extensively infiltrated tumors.

Unsupervised cluster analysis revealed that the greatest differences reflected the tissue examined rather than the individual patient, with patient and donor blood segregating together. The gene expression profiles were analyzed in a variety of comparisons, including TIL-vs-LN or P-PB, P-PB-vs-D-PB and ER+-vs-ER- tumors. The extent of the CD4+ T-cell infiltrate determined by IHC revealed that 2 ER+ and 2 ER- tumors were extensively-infiltrated while 3 ER+ and 3 ER- were minimally-infiltrated with these groups also compared.

The inventors conclude that the CD4+ T-cell profiles: 1) were remarkably similar between P-PB and D-PB, with the autologous cells constituting a good internal control; 2) found surprisingly few gene changes were detected between ER+ and ER- tumors; and 3) detected important differences in immune response genes between extensive and minimally-infiltrated tumors (extensively-infiltrated defined as > 5% intra-tumoral and > 10% intra-stromal lymphocytes), moderately-infiltrated defined as > 5% intra-tumoral or >10% intra-stromal lymphocyte, and minimally infiltrated defined as < 5% intra-tumoral and < 10% intra-stromal lymphocyte.

CD4+ helper T (Th) subsets present in the TIL were assessed by comparing patient data with publically available Th cell gene expression microarray datasets. The public datasets extend each Th profile beyond conventional markers and overall are relatively specific for a given subpopulation.

A direct comparison of patient CD4+ T-cell data with the public Th gene lists produced individual profiles comprised of independent gene sets showing that:
1) D-PB and P-PB are relatively similar across all Th subsets, with a slight increase in effector memory cell markers in P-PB;
2) LN profiles are transitional between P-PB and TIL, with upregulation of the Th1 genes CD38, CXCL9, IFNG and IL2, the Tfh genes CD200, CXCL13 and ICOS, and the transcription factors FOS, FOSB, JUN, JUNB, JUND and REL notable among the limited number of altered immune response genes detected (LN-vs-P-PB);
3) the TIL profile is enriched in all major Th subpopulations (Th1, Th2, Tfh, Treg and Th17);
4) the TIL are activated CD25+ cells; and
5) the TIL have a memory phenotype closer to effector than central memory cells. Expression levels of conventional Th markers reiterate that all Th subpopulations are present in the TIL but do not provide information on their relative distribution.

Alternatively, the comparison of TIL from extensive versus minimally-infiltrated tumors revealed a distinct skew to Th1 (CD38, CXCL9, CXCL10, CXCL11, IFNG and LTB) and Tfh (ICOS, CD200, CXCL13 and PDCD1) gene expression in the extensively-infiltrated tumors.

Flow cytometric analysis of CD4+ TIL from BC patients compared with CD4+ T-cells from healthy donor blood largely validated these data and provided more quantitative information, demonstrating that the CD4+ TIL are antigen-experienced effector memory cells (CD45RO+) characterized either wholly or in a subpopulation of cells as: TCR/CD3^{lo} CD6^{lo} CD7^{lo} CD11b+ CD18+ CD24+ CD25± CD26± CD27^{lo} CD28^{lo} CD35- CD38^{±} CD43^{lo} CD44+ CD49D^{±} CD54^{hi} CD55^{lo} CD57^{lo} CD58^{hi} CD59+ CD62L^{lo} CD69+ CD71+ CD73+ CD80+ CD86+ CD109+ CD146+ CD166+ CD200^{±} CCR4+ CCR5+ CCR6^{lo} CCR7^{lo} CX3CR^{lo} CXCR3+ CXCR4+ CXCR5+ 4-1BB+ BTLA+ CRTH2+ CTLA4+ FAS^{hi} GITR+ HLA-DR+ ICOS+ IL1R1+ IL2RB+ IL6ST^{lo} IL7R^{±} IL12RB2+ IL17RB+ IL17RD+ OX40+ OX40L+ PD-1+ TGFBR2+ TGFBR3+. The heterogeneity observed in some activation-induced markers (ex. CD25, CD71, HLA-DR) suggests that not all of the TIL are activated.

Upregulation of conventional Th surface markers on CD4+ TIL subpopulations was detected and included:
1) the Th1 markers CD38, CCR5, CD54 and IL12RB2;
2) the Th2 marker CCR4;
3) the Th17 marker IL1R1;
4) the Tfh markers BTLA, CD200, ICOS and PD-1; and
5) the Treg markers CD25 and GITR.
Interestingly, surface expression of the Th markers CD38, CD58, CD200, GITR, ICOS and PD-1 was increased on CD4+ TIL from extensive relative to minimally-infiltrated tumors.

Many of the gene and surface markers expressed by the TIL reflect an activated state; however, only low levels of Th-derived cytokines/chemokines were statistically significantly increased in the TIL-vs-P-PB comparison (upregulated genes: CXCL9, CXCL10, CXCL11, CXCL13, IL6, IL18, IFNG, and XCL1).

Follow-up using more sensitive qRT-PCR confirmed these increases and expanded the set of Th-derived factors [Th1 (CXCL9, CXCL10, CXCL11, GZMA, GZMB, IFNG, IL2, IL18 and LTB), Th2 (IL13), Th17 (CCL20, IL6, IL17A, IL17F, IL21 and IL22), Tfh (CXCL13 and IL21) and Treg (IL10, TGFβ and XCL1)], although many were not detected at activation-induced levels (microarray data comparisons), with lower expression more pronounced in minimal relative to extensively-infiltrated tumors. These data indicate that while all Th subsets are infiltrating BC, the full complement of Th cytokines and chemokines is not produced.

In addition, the inventors have found that specific B lymphocytes are present in the tumor in case of an organized immune response. More particularly, the inventors have identified Bm3 and/or Bm4 lymphocytes (in addition to Bm1 Bm2, and Bm5 lymphocytes).

In the context of the present invention, centrocytes or Bm4 lymphocytes are negative for IgD expression, but express high levels of CD38 protein. Centroblasts or Bm3 lymphocytes are negative for IgD expression, but express high amounts of CD38 protein. Bm1 are negative for CD38 and Bm2 express intermediate levels of CD38 in humans. Bm5 are negative for IgD and CD38.

The inventors have then investigated the nature of the activation state of CD4+ TIL. Among the downregulated genes in the TIL compared to their counterparts from LN or P-PB are components of the TCR/CD3 complex, molecules involved in signal transduction, co-stimulatory receptors, transcription factors and negative regulatory molecules. Some of these gene changes were confirmed at the mRNA or protein level.

A comparison with gene expression profiles of anti-CD3/CD28 stimulated donor CD4+CD45RO+ T-cells revealed that approximately half of these changes were similarly induced or repressed as observed with TCR/CD3 activation.

TCR/CD3 downregulation on TIL has been described for a wide variety of solid tumors, but the depth of this suppression, extending through the signaling pathway and divergent from activation-induced modifications, has not been previously reported. The disparity within T-cell activation is highlighted by the number of aberrantly expressed co-receptors, which are involved in regulating the TCR/CD3 signal.

Interestingly, the NF-κB transcription factor REL and members of the AP-1 family (ATF3, BATF, FOSB/FOSL2 and JUN/JUND) were specifically upregulated despite reduced upstream MAPK signaling. The significance of this pattern of TCR/CD3 signaling gene expression is supported by the differences observed in extensive versus minimally-infiltrated tumors.

Extensively-infiltrated tumors are characterized by higher expression of the TCR/CD3 subunits and numerous co-receptor genes, including CD38, CD200, ICOS, PD-1 (PDCD1), as well as some signaling genes and transcription factors. These data suggest that extensively-infiltrated tumors are characterized by lower levels of TCR/CD3 suppression in concert with higher levels of activation.

The inventors next performed a series of *in vitro* experiments to explore this combination of activation and suppression. CD4+ (or CD4+CD45RO+) T-cells from D-PB were treated with supernatants (SN) derived from fresh tumor homogenates, alone or in conjunction with anti-CD3/CD28 stimulation (S).

SN downregulated TCR/CD3 expression and inhibited activation-induced surface receptor expression, characterized by CD69, ICOS, CXCR5, CD25 and FAS. Alternatively, SN and to a lesser extent SN+S induced expression of CXCR4, a receptor for the chemokine CXCL12 whose pro-tumor activities are well documented.

Global gene expression arrays revealed that about 75% of the genes commonly altered in the TIL and their SN-treated donor PB counterparts were either similarly up- or downregulated.

Expression profiles of the donor-treated cells (versus untreated controls) were compared with the TIL-vs-P-PB data, clearly demonstrating that the SN effect is significantly amplified by activation, with the SN+S cells more closely resembling the TIL than SN alone. The SN+S gene profiles are also distinct from S alone and largely reiterate this mixture of activation and suppression.
Examination of the SN+S effect on TCR/CD3 pathway genes detected:
1) significant suppression of various signaling genes to a greater extent than the S donor memory cells;
2) specific upregulation of REL and AP-1 family transcription factors similar to the TIL;
3) greater suppression of the affected co-stimulatory receptor genes compared with the TIL or S-treated donor cells; and
4) a similar pattern of negative regulatory gene expression between the SN+S and S-treated cells.

The SN+S-treated cells suppressed most of the activation-induced changes detected in their S counterparts, including the majority of cytokines or chemokines. Among the Th cytokines/chemokines produced in the S donor cells, only the pro-inflammatory, tumor-promoting factors CCL20, IL-8 and TNF are further upregulated in SN+S-treated cells. IFNγ and the IFNγ-induced chemokines CXCL9, CXCL10 and CXCL11 are the only S-induced factors also expressed in the TIL, with their overt suppression by SN+S perhaps reflecting a stronger effect from the high local concentrations generated *in vitro.* This is supported by the observed SN-mediated suppression of CXCR3 (CXCL9, CXCL10, CXCL11 receptor) in parallel with CXCR4 upregulation in NS or S donor cells.

Overall, these data consistently support the view that the simultaneously activated/suppressed TIL profile is broadly reproduced in the SN+S donor CD4+ T-cells, where the suppressive SN effect is amplified by T-cell activation.

To assess whether these tumor-mediated effects on CD4+ T-cells were transient, freshly isolated TIL were split and either extracted immediately or "rested" *ex vivo* overnight without stimulation. Gene expression profiling shows that the rested TIL reverse expression in a significant number of genes expressed in their freshly isolated counterparts. Notable are the cytokine/chemokine genes, including IFNγ and IFNγ-induced genes, which are upregulated in the TIL and return to baseline 24h after removal from the tumor microenvironment. The B-cell chemoattractant CXCL13 levels (associated to extensive TIL) decreased only by half at 24h.

Reversal of TCR/CD3-directed pathway gene expression in the rested TIL was limited to BTLA and the AP-1 factors ATF3, FOSB and JUN, indicating that suppression of TCR/CD3 signaling has not been reversed at 24h.

These experiments show that the immunosuppressive quality of individual BCs varies, with a tendency for tumors sustaining an extensive lymphocyte infiltrate to be less immunosuppressive than minimally-infiltrated tumors.

A direct comparison of gene expression in TIL from extensive versus minimally-infiltrated tumors supports this conclusion by showing that extensively-infiltrated tumors are most similar to S-treated donor cells.

The inventors then examined the specific genes involved. These analyses revealed that a variety of immune response genes are expressed at elevated levels in TIL from extensively-infiltrated BC.

The most distinctive increase consistently detected in extensively-infiltrated tumors was the CXCL13 gene with CD4+ TIL determined to be the principal cellular source. CXCL13 is a chemokine for CD4+ Tfh and B-cell recruitment to LN. CXCR5 (CXCL13 receptor) expression was reduced by SN. Comparison of CXCL13 with the expression of other cytokines and chemokines in an extensive and minimally-infiltrated tumor revealed that the majority are expressed by infiltrating leukocytes (CD14+ monocytes, CD4+ T-cells or the remaining mixture of CD45+ cells) but not the EpCAM+ tumor cells.

Except the pro-tumor factor CXCL12, anti-tumor cytokines and chemokines are expressed at significantly higher levels in the extensive compared to the minimally-infiltrated tumor.

CD4+ T-cells and CD14+ monocytes produce the proinflammatory cytokine IL-18, (IFNγ-induced) CXCL9, CXCL10 & CXCL11 while IFNG originated from the remaining CD45+ cells, presumably produced by CD8+ T-cells.

To verify these observations, qRT-PCR data and lymphocyte infiltration intensity from 21 tumors were analyzed to produce a correlation plot and generate unsupervised hierarchical clustering. This analysis shows that expression of the IFNG, CXCL9, CXCL10, CXCL11 and CXCL13 genes is correlated with CD38, CD58, ICOS, PDCD1 (PD-1) and CD200 gene expression in CD4+ TIL. Furthermore, these genes are also significantly correlated with lymphocyte infiltration and more specifically with CD8+ T-cell and B-cell marker expression in the non-CD4+ tumor fraction.

The Th marker genes were negatively correlated with EPCAM in the non-CD4+ tumor fraction. Flow cytometry confirmed that higher numbers of CD4+ TIL expressing surface CD38, CD200, ICOS and PD-1 were present in extensively-infiltrated tumors. The principal CXCL13-producing cells were identified as PD-1^{hi}CD26^{lo}CD38^{±}CD200^{hi}CD4+TIL.

Thus, emerging from these analyses was evidence that in the CD4+ fraction, a Th1 profile (CD38, IFNG and CXCL9) was associated with a Tfh profile (CD200, PD-1, ICOS and CXCL13) and both with more extensive lymphocyte infiltration, suggesting these tumors are skewed not only to Th1 but also to Tfh CD4+ T-cells.

The inventors then analyzed BC for lymphoid aggregates and organized structures such as tertiary lymphoid structures (TLS) with or without a detectable germinal center (GC). TLS (and GC) were detected adjacent to tumor nests at a higher incidence in extensive compared to minimally-infiltrated tumors. The intensity and distribution of the major CD45+ TIL subpopulations within the TLS and GC were further assessed using IHC. BC TLS organization was found to be similar to LNs, with a CD3+ T-cell zone adjacent to a CD20+ B-cell zone and CD23+ FDC, Ki67+ and Bcl6+ (a marker for Tfh and GC B-cells) cells present exclusively in the GC.

CD4+ T-cells are the principal component of the T-cell zone and also have a significant presence in the GC while CD8+ T-cells are moderately interspersed with CD4+ T-cells in the T-cell zone and absent from the GC.

CXCL13 was principally expressed by T-cell zone lymphocytes and GC lymphocytes, and FDCs, with intratumoral CXCL13+ lymphocytes detected in some extensively-infiltrated tumors.

TLS and GC thus function to more efficiently organize effective anti-tumor immune responses adjacent to the tumor while providing a refuge from rapid tumor-mediated immunosuppression.

The clinical relevance to BC outcome of a Tfh gene expression profile (as a reflection of a TLS presence) was investigated to test this hypothesis by deriving signatures reflecting important differences between extensive and minimally-infiltrated tumors. The prognostic power of the signatures of the present invention for relapse-free survival was compared with the STAT1 immune signature (Desmedt et al., Ignatiadis et al.) on a set of 794 untreated primary BC. Similar to STAT1, the present (Th1) signature (14 genes; CCND2, CD38, CTLA4, EED, PMAIP1, RAB27A; SH2D2A, STAT1, TNFRSF4, IL12RB2, SAMD9L, CXCL9 and IFNγ) predicted survival in the HER2+ BC (P=0.005) subset alone. The Tfh signature (8 genes; CD200, CXCL13, FBLN7, ICOS, SGPP2, SH2D1A, TIGIT, PD1) was the best predictor for patient outcome, predicting survival not only in the HER2+ (P=0.012) and ER+/HER2- high proliferative (P=0.025) subtypes but also in the overall patient set (P=0.0036).

The Tfh signature also had a tendency to be prognostic (P=0.082) for the ER-/HER2- subtype.

The eight genes in the Tfh signature were tested individually with CXCL13 the best predictor for overall patient survival (P=0.0046).

The expression level of CD200 revealed different features. When measured on a whole tumor sample, high expression levels are correlated with increased survival of ER+(HER2-) BC patients. However, in ER- HER2-BC patients, high expression levels measured on a whole tumor sample, are correlated with decreased survival.
On the other hand, co-labelling (for instance by FACS sorting) of CD200 protein with lymphocyte specific markers, such as CD4, adequately addresses the Tfh TIL population within a tumor (tumor cells may also express CD200 protein): increased levels of these cells represent increased overall survival in cancer patients or reflect a good response to pre-operative chemotherapy.

These results indicate that the number of CD4+ Tfh TIL required for recruiting other immune cells to the tumor, initiating TLS/GC formation to establish a protected microenvironment, and providing CD4+ T-cell help for effective memory anti-tumor immune responses is relatively small compared with other immune effector subpopulations.

Material and methods

Tumor, lymph node and/or blood samples were obtained from 70 untreated patients undergoing tumor resection for breast cancer at the Jules Bordet Institute. Fresh tumor tissue, lymph node tissue and peripheral blood from the ten patients in the discovery set (P1-P10) and fresh tumor tissue from 60 patients in the confirmation set (TIL003-TIL132; with ipsilateral non-adjacent normal breast tissue from 31 patients) were analyzed. Peripheral blood samples from 19 healthy female adults were used as controls for the discovery set (D1-4) and the confirmation set (D5-19). All specimens were acquired using a protocol approved by the local ethics committee with written informed consent obtained from each patient.

Tumor sample preparation

Dissected tumor fragments from fresh surgical specimens were directly transferred into 3ml of X-VIVO 20 (Lonza) before two rapid rounds of mechanical dissociation with the GentleMACS™ Dissociator (Program A.01 both rounds; Myltenyi Biotec). The resulting cell suspension was filtered following each dissociator run using a 40µm cell strainer (BD Falcon), washed with X-VIVO 20, centrifuged 15min at 600g, and resuspended in X-VIVO 20 before isolation or flow cytometric analysis. The tumor SN was the initial 3ml of X-VIVO 20 recovered after the first round of dissociation, which was subsequently clarified by centrifugation for 15 min at 13000xg.

Cell isolation

For molecular analyses, CD4+ T-cells were positively purified from patient samples or healthy donor blood using Dynabeads^{®} CD4 (Invitrogen). Additional tumor cell populations were purified using Dynabeads^{®} M-450 Epoxy (Invitrogen) coupled with monoclonal antibodies to CD14, CD45 or EpCAM (#301801, #304001 and #324201, Biolegend) followed by Dynabeads^{®} CD4 positive selection. Memory CD4+ cells were purified by CD45RA depletion [Dynabeads^{®} M-450 Epoxy coupled to anti-CD45RA (#304102, Biolegend). For primary cells treated with tumor SN, total CD4+ or memory CD4+ cells were negatively purified using the CD4+ T-cell Isolation Kit II or Memory CD4+ T-cell Isolation Kit (Myltenyi Biotec). The cell purification protocols were controlled by flow cytometry with negatively purified samples >98% pure and positively isolated CD4+ TIL >95% pure.

Treatment with tumor supernatant

Total CD4+ or memory CD4+ cells (2x10⁶ cells/ml) purified from healthy donor blood were incubated in X-VIVO 20 overnight following isolation. Cell viability was controlled the following day by flow cytometry (>95%) and then an equal volume of tumor SN or X-VIVO 20 was added ± anti-CD3 (#317304, clone OKT3; Biolegend) and anti-CD28 (#555725, clone CD28.2; BD Pharmingen), each at 1µg/ml. The cells were recovered 24h after treatment for analysis using microarrays, qRT-PCR (including an experiment to establish the kinetics of IFNG and IL2 gene expression after treatment up to 48h; Table S4h) and/or flow cytometry.

Microarrays

10ng (discovery set) or 100ng (confirmation set) of high quality total RNA was labeled following the manufacturer's protocols for probe preparation and hybridization on the Affymetrix U133 Plus 2.0 GeneChip^{™}.

Quantitative PCR with reverse transcription (qRT-PCR)

RNA was extracted using TRIzol Reagent (Invitrogen) and reverse transcribed into cDNA using High Capacity RNA-to-cDNA (Applied Biosystems) following standard procedures. Real time PCR reactions were performed using commercially available Gene Expression Assays (Applied Biosystems or Qiagen) or published primers (detailed with references in Table S4a) and iTaq™ SYBR® Green Supermix With ROX (Bio-Rad) on an ABI 7900HT Prism sequence detector (Applied Biosystems).

p-values for non-microarray data were generated using a two-tailed student t-test with unequal variance. Individual microarray data sets for patient data, experimental data and publically available data for individual Th subsets were differentially analyzed based on the type and number of samples in the set by using specifically adapted methods and criteria.

Flow cytometry

Samples assessed for the expression of 60 cell surface markers (duplicates or triplicates were assessed for each marker) were incubated individually or in combinations of antibodies (listed in Figures S1-S3) for 45min at 4C in 50ul of X-VIVO 20 followed by washing with 2ml of PBS and immediate analysis on a FACS Calibur (BD Biosciences) without fixation. Cytofix/Cytoperm™ kit (BD Biosciences) was used for intracellular staining of anti-CXCL13 (R&D Systems, IC801A).

Immunohistochemistry

Four µm sections of paraffin-embedded tumor blocs from P1-10 were stained with anti-CD4 and anti-CD3 antibodies. Successive sections from 15 patients of the confirmation set were stained with the antibodies listed in Figure S4 using Endogenous Biotin Blocking Kit (Ventana) and iVIEW DAB Detection Kit (Ventana) on a BenchMark XT IHC/ISH slide stainer (Ventana). For CXCL13 (R&D Systems, AF801, 1/100) staining, mounted sections were deparaffinized with xylene, pretreated with citrate 10mM pH 6.0 at 95 to 99°C for 20 min, exposed to 3% hydrogen peroxide solution (Sigma Aldrich) to quench endogenous peroxidase activity, blocked with rabbit serum (Sigma) for 1 h before incubation with anti-CXCL13 in a moist chamber at 4°C overnight. A negative control section, without the primary antibody, was run in parallel for each slide. Subsequently, after wasting a biotinylated rabbit anti-goat secondary antibody (Vector Laboratories) was applied for 30 min at room temperature, followed by the HRP streptavidin reagent (Zymed) and the DAB + Substrate Chromogen System (Dako). The slides were finally counterstained with hematoxylin (Sigma-Aldrich) and mounted on the Tissue-Tek® Prisma™ Automated Slide Stainer (Sakura).

IHC slides stained for CD3 and CD4 were read by a pathologist to determine the extent of T-cell infiltration in tumors from the discovery set, identifying extensive (n=4) and minimally-infiltrated (n=6) tumors. H&E slides were independently read by a pathologist and a biologist to determine the extent of intra- and peritumoral lymphocyte infiltrates, the number of TLS/lymphoid aggregates and GC present in tumors from the confirmation set, identifying extensive (n=15), moderate (n=5) and minimally-infiltrated (n=37) tumors. Tumors from three patients did undeterminable. Lymphocyte infiltration levels and the presence of lymphoid structures were correlated with patient clinico-pathological parameters and the results are shown in Table S1c. IHC staining for leukocyte markers in tumors from 15 confirmation set patients were read and analyzed by a biologist and reviewed independently by a pathologist.

Survival analyses assessing the STAT1 (Desmedt et al.), Th1 and Tfh gene signatures or individual Tfh genes were performed using 794 untreated BC patients from 8 public microarray data sets of untreated patients). Three categories of gene expression cut off values (high, intermediate and low for each signature) were defined using tertiles of the continuous signature scores (= mean intensity value of all genes within a given signature). BC subtypes are defined using a two-gene classification based on ESR1 (ER) and ERBB2 (HER2) gene expression.

## Claims

1. A diagnostic kit comprising tools for measuring CXCL13 gene expression, and tools for measuring the expression of a (1, 2, 3, 4, 5, 6, or all the 7) gene(s) selected from the group consisting of CD200, FBLN7, ICOS, SGPP2, SH2D1A, TIGIT and PDCD1.

2. The kit of claim 1 comprising antibodies specifically recognizing one or more (1, 2, 3 or all the 4) of the proteins encoded by the gene(s) selected from the group consisting of CD200, ICOS, PDCD1 and TIGIT gene(s) and further comprising antibodies specifically recognizing CD4 protein.

3. The kit of claims 1 or 2 further comprising tools for measuring the expression of genes selected from the group consisting of CXCL9, IFNG, PMAIP1, IL12RB2, CD38 and CTLA4 gene(s).

4. An isolated human CD4-positive lymphocyte having high expression level of both PDCD1 and of CD200 proteins.

5. The lymphocyte of claim 4 having further low or no expression of CD26 protein.

6. A method for prognosticating the outcome of a solid-tumor cancer selected from the group consisting of breast cancer, pancreatic cancer, prostate cancer, colorectal cancer, lung cancer, hepatocellular carcinoma and renal cell carcinoma, preferably breast cancer, comprising the step of
measuring the expression on a whole tumor sample of CXCL13 gene and of a (1, 2, 3, 4, 5, 6, or all the 7) gene(s) selected from the group consisting of CD200, FBLN7, ICOS, SGPP2, SH2D1A, TIGIT and PDCD1; and/or of
assessing the presence of tissular lymphoid structures containing specific B and specific T cells, wherein the said specific B and T cells comprise CD4-positive follicular helper T cells (Tfh; such as the lymphocyte of claims 4 or 5) and/or Bm3 and/or Bm4 B cells within a tumor sample from the said solid-tumor cancer; and/or of homogeneizing in vitro a tumor sample; performing a centrifugation of the said homogenized sample; applying the obtained supernatant to a culture of CD4+ reporter cells and measuring the effects on proliferation of the said CD4 positive cells in response to stimulation.

7. The method of claim 6, wherein overexpression of CXCL13 gene and of a (1, 2, 3, 4, 5, 6, or all the 7) gene(s) selected from the group consisting of CD200, FBLN7, ICOS, SGPP2, SH2D1A, TIGIT and PDCD1 in the tumor sample is associated with a good prognostic.

8. The method of claim 7, wherein the expression of CD200, ICOS, PDCD1 and/or TIGIT genes is measured in flow cytometry on CD4-positive lymphocytes freshly isolated from the tumor sample.

9. The method according to any of the preceding claims 6 to 8, wherein the expression of CD200 gene is measured in dual labelling with PDCD1 protein.

10. The method according to any of the preceding claims 6 to 9, wherein the expression level of the genes is measured by comparison with the gene expression level of CD4+ cells from an *ex vivo* sample of peripheral blood from the patient affected with the solid tumor cancer.

11. A method for measuring primary tumor aggressivity and/or for identifying the presence of immunosuppressant factors in tumors comprising the steps of:
- homogeneizing *in vitro* a tumor sample,
- performing a centrifugation of the said homogenized sample,
- applying the obtained supernatant to a culture of CD4+ reporter cells and
- measuring the effects on proliferation of the said CD4-positive cells upon stimulation,
wherein the said primary tumor is from a cancer selected from the group consisting of breast cancer, pancreatic cancer, prostate cancer, colorectal cancer, lung cancer, hepatocellular carcinoma and renal cell carcinoma, preferably breast cancer.

12. The method of claim 11 further comprising the step of adding anti-CD3 and/or anti-CD28 antibodies to the obtained supernatant to be applied to the culture of CD4+ reporter cells.

13. CXCL13 protein for use in the treatment of solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein a low (or no) expression of the genes and/or no tissular lymphoid structure and/or impaired growth of CD4+ reporter cells is measured by the method according to any of the preceding claims 6 to 10.

14. Anti PD1 (PDCD1) antibodies for use in the treatment of a solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein a moderate to high expression of the genes and/or tissular lymphoid structures and/or less impaired growth and responsiveness of CD4+ reporter cells is measured by the method according to any of the preceding claims 6 to 10.

15. Anti CTLA4 antibodies for use in the treatment of a solid tumor cancer, selected from the group consisting of breast cancer, pancreatic cancer, prostate cancer, colorectal cancer, lung cancer, hepatocellular carcinoma and renal cell carcinoma, the said cancer affecting a patient wherein moderate to high expression of the genes and/or tissular lymphoid structure and/or less impaired growth and responsiveness of CD4+ reporter cells is measured by the method according to any of the preceding claims 6 to 10.
